# EUROPEAN PATENT APPLICATION

(11) **EP 4 484 552 A1**
(43) Date of publication of application: **01.01.2025**
(21) Application number: 23756501.5
(22) Date of filing: 21.02.2023
(51) Int. Cl.: C12N 5/07, C12N 7/00, C12N 15/64

(54) **VIRAL VECTOR PRODUCTIVITY ENHANCER AND PRODUCTION METHOD OF VIRAL VECTOR**

(30) Priority: 21.02.2022 JP 2022025036
(71) Applicant: NIPPON MEDICAL SCHOOL FOUNDATION, Bunkyo-ku Tokyo 113-8602 (JP)
(72) Inventor: MIYAGAWA Yoshitaka, Tokyo 113-8602 (JP); OKADA Takashi, Tokyo 113-8602 (JP); KOBARI Yukage, Tokyo 113-8602 (JP); UCHIKAI Takao, Tokyo 104-0042 (JP)
(74) Representative: Mewburn Ellis LLP
(86) International application number: PCT/JP2023/006198
(87) International publication number: WO 2023/157977

(57) **Abstract**

This invention is intended to enhance production of viral vectors when producing viral vectors using viral vector-producing cells. DNA replication in viral vector-producing cells is inhibited.

## Description

### Technical Field

The present invention relates to an agent for enhancing production of viral vectors capable of enhancing production of viruses, which can be used in a process for producing viral vectors using cultured cells, and a method for producing viral vectors.

### Background Art

Gene therapy involving the use of viral vectors is a method of treatment comprising administering a viral vector comprising, integrated therein, a gene encoding a therapeutically effective protein to a patient or administering a cell comprising, introduced thereinto, such viral vector to a patient. Examples of viral vectors used in gene therapy include retrovirus, lentivirus, adenovirus, adeno-associated virus, Sendai virus, and herpesvirus vectors. While practical application of gene therapy has begun to date, there is an urgent need to develop a technique that enables efficient large-scale production of high-quality viral vectors in order to realize extensive application of gene therapy to a wide variety of diseases.

In general, viral vectors are produced by performing culture to proliferate vector-producing cells and then infecting the cells with a small amount of viruses. Thereafter, cell culture is continued to expand viral infection, and viral vectors are then proliferated in the virus-infected cells. The viral vectors proliferated in the cells are collected and purified in accordance with conventional techniques and then formulated, according to need.

In such a process of producing viral vectors, various attempts have been made in order to enhance production of viral vectors. For example, Patent Literature 1 discloses an agent that can improve efficiency for introducing a recombinant adeno-associated virus into a cell (i.e., transfection efficiency) in the process of producing viral vectors. The enhancing agent disclosed in Patent Literature 1 is valproic acid, a salt or a derivative thereof, isobutyric acid, a salt or a derivative thereof, or isovaleric acid, a salt or a derivative thereof.

Patent Literature 2 discloses an invention concerning a cell line used in a process of production of an adeno-associated viral vector in which expression of at least one of a gene encoding YB1 (also known as the Y box binding protein 1, the Y box transcription factor, or the nuclease sensitive element binding protein 1), a gene encoding NPM1 (also known as the nucleophosmin, the nucleolar phosphoprotein B23, or numatrin), and a gene encoding NCL (the nucleolar phosphoprotein nucleolin) is decreased. Use of the cell line disclosed in Patent Literature 2 leads to enhanced production of viral vectors.

In addition, Patent Literature 3 discloses an invention concerning a cell line used in a process of producing an adeno-associated viral vector into which a particular miRNA has been introduced. Use of the cell line disclosed in Patent Literature 3 enables production of adeno-associated viral vectors of higher titers than conventional viral vectors without complicated procedures.

### Citation List

### Patent Literature

Patent Literature 1: JP 2020-524498 A
Patent Literature 2: JP 2017-506885 A
Patent Literature 3: JP Patent No. 6,093,358

### Summary of Invention

### Technical Problem

The known techniques indicated above aimed at enhancing production of viral vectors; however, were not sufficient from the viewpoint of production of viral vectors. Accordingly, it remains necessary to develop a technique aimed at enhancing production of viral vectors.

Under the above circumstances, it is an object of the present invention to provide a novel agent for enhancing production of viral vectors that can enhance production of viral vectors and a method for producing viral vectors.

### Solution to Problem

The present inventors have conducted concentrated studies in order to attain the object described above. As a result, they discovered that production of viral vectors would be enhanced by inhibiting DNA replication in viral vector-producing cells. This has led to the completion of the present invention. The present invention encompasses the following.

(1) An agent for enhancing production of viral vectors comprising, as a major ingredient, a DNA replication inhibitor.
(2) The agent for enhancing production of viral vectors according to (1), which is used for production of viral vectors using viral vector-producing cells.
(3) The agent for enhancing production of viral vectors according to (1), wherein the DNA replication inhibitor is a platinum-based drug.
(4) A method for producing viral vectors comprising: a step of culturing viral vector-producing cells in which DNA replication is inhibited; and a step of collecting viral vectors from the cultured cells.
(5) The method for producing viral vectors according to (4), wherein, in the step of culturing cells, the cells are cultured in a medium supplemented with the agent for enhancing production of viral vectors according to any of (1) to (3).

This description includes part or all of the content as disclosed in the description and/or drawings of Japanese Patent Application No. 2022-025036, which is a priority document of the present application.

### Advantageous Effects of Invention

The present invention can provide an agent for enhancing production of viral vectors that can act on viral vector-producing cells to significantly enhance production of viral vectors from the cells. The present invention can also provide a method capable of significantly enhancing production of viral vectors, in a production of viral vectors using viral vector-producing cells.

### Brief Description of Drawings

[Figure 1] Figure 1 shows a chart demonstrating the results of measuring the effects of enhancing production of rAAV vectors achieved with the use of Carboplatin.
[Figure 2] Figure 2 shows a chart demonstrating the results of measuring the effects of enhancing production of rAAV vectors achieved with the use of Carboplatin with the elapse of time.
[Figure 3] Figure 3 shows an electrophoretic image demonstrating the results of the SDS-PAGE analysis of rAAV vector-constituting proteins when Carboplatin is used.
[Figure 4] Figure 4 shows photographs demonstrating the results of the infectivity test of the rAAV vectors produced with the use of Carboplatin.
[Figure 5] Figure 5 shows a chart demonstrating the results of measuring the effects of enhancing production of rAAV vectors achieved with the use of Cisplatin.
[Figure 6] Figure 6 shows a chart demonstrating the results of measuring the effects of enhancing production of rAAV vectors achieved with the use of Nedaplatin.

### Description of Embodiments

The agent for enhancing production of viral vectors and the method for producing viral vectors according to the present invention enhance production of viral vectors using viral vector-producing cells.

In an embodiment of the present invention, DNA replication in viral vector-producing cells is inhibited, so as to enhance production of viral vectors. The term "production of viral vectors" used herein indicates that the amount of viral vectors is larger than that of the control when the cells are cultured under given conditions or the term indicates that the virus titer is higher than that of the control when the cells are cultured under given conditions. The amount of viral vectors or the virus titer can be adequately measured in accordance with conventional techniques. Examples of techniques for measuring the amount of viral vectors or the virus titer include, but are not particularly limited to, plaque assay, TCID50 assay (the tissue culture infectious dose), and quantitative RT-PCR assay.

In the present invention, viral vectors are viruses produced and amplified by artificial means and genetically modified viruses for research and medical purposes. Types of viruses are not particularly limited, and examples of viruses include: non-enveloped viruses, such as adeno-associated virus (AAV), adenovirus, enterovirus, parvovirus, papovavirus, human papillomavirus, rotavirus, Coxsackie virus, Sapovirus, norovirus, poliovirus, echovirus, hepatitis A virus, hepatitis E virus, rhinovirus, astrovirus, circovirus, and simian virus; and enveloped viruses, such as retrovirus, lentivirus, Sendai virus, herpesvirus such as herpes simplex virus, vaccinia virus, measles virus, baculovirus, influenza virus, leukemia virus, Sindbis virus, and poxvirus. Among them, a viral vector used for gene therapy, such as the adeno-associated virus, is preferably used.

An adeno-associated virus is a member of the Parvoviridae virus family that packages a linear single-stranded DNA in a capsid. Examples of adeno-associated viruses include AAV type 1 (AAV1), AAV type 2 (AAV2), AAV type 3 (AAV3), AAV type 4 (AAV4), AAV type 5 (AAV5), AAV type 6 (AAV6), AAV type 7 (AAV7), AAV type 8 (AAV8), AAV type 9 (AAV9), and AAV type 10 (AAV10). Examples also include artificially prepared AAV viral vectors, such as AAV-DJ and AAV-PHP.B. Such viral vectors can be applied to techniques for packaging and amplifying artificial nucleic acids in adeno-associated viral capsids.

The term "viral vector-producing cell" is synonymous with a packaging cell that is capable of producing the viral vectors. An example thereof is a cell resulting from transfection of a viral vector into the human embryonic kidney 293 (HEK293) cell (i.e., a packaging cell). A cell to be transfected with a viral vector is not limited to the HEK293 cell, and examples thereof include HEK293T cell, HEK293S cell, HEK293F cell, HEK293FT cell, HEK293FTM cell, HEK293 SG cell, HEK293 SGGD cell, HEK293H cell, HEK293E cell, and HEK293MSR cell derived from the HEK293 cell. A commercially available cell developed for the adenovirus (e.g., the Adeno-X 293 cell line), a commercially available cell developed for the adeno-associated virus (e.g., the AAVpro 293T cell line), and the like can also be used. Examples of other mammal-derived packaging cells include human cervical cancer-derived HeLa cell, human lung cancer-derived A549 cell, human fibrosarcoma HT-1080 cell, human retinal pigment epithelium-derived cell, such as the PER. C6 cell, hamster-derived BHK and CHO cells, and African green monkey-derived Vero and COS cells.

A technique of producing viral vectors is not limited to a method for transfecting plasmids into packaging cells, and examples of techniques include a method of infecting packaging cells with helper viruses, such as adenoviruses or herpesviruses, to amplify the viruses and a method of integrating a constitutive factor for packaging viral vectors into the cell genome to establish and amplify producer cells.

Viral vector-producing cells can be used for techniques that amplify viral vectors using insect-derived cells in addition to mammal-derived cells. Examples of such techniques include a method of amplification comprising transfection of viral vectors with the use of lepidopteran insect-derived cells represented by Sf9 and Sf21 cells derived from the ovary of the cabbage armyworm and Tni cells and High Five cells derived from *Trichoplusia ni,* and a method of amplification comprising infection of the cells with the baculovirus comprising a constitutive factor for packaging viral vectors mounted thereon.

A medium in which the viral vector-producing cells are cultured is not particularly limited, and an adequate medium can be selected in accordance with the cells to be used. Examples of media that can be used include the Dulbecco's Modified Eagle Medium supplemented with 10% fetal bovine serum (DMEM supplemented with 10% FBS or DMEM supplemented with 10% FCS) and the serum-free Eagle Minimum Essential Medium (E-MEM). Viral vector-producing cells may be cultured by adhesion culture or suspension culture.

### [Inhibition of DNA replication]

In the present invention, the term "inhibition of DNA replication" indicates, but is not particularly limited to, that a DNA replication inhibitor, such as a platinum-based drug, is allowed to act on the viral vector-producing cells. Examples of platinum-based drugs that can be used in the present invention include, but are not particularly limited to, Carboplatin, Oxaliplatin, Cisplatin, and Nedaplatin and analogs of such compounds. As the DNA replication inhibitor, in particular, use of Carboplatin having the structure represented by the structural formula below or an analog thereof is preferable.

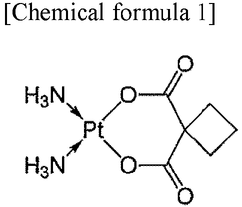

An analog of Carboplatin is a compound having a structure derived from the structure indicated above by substitution of a given atom or atoms with another atom or other atoms, which binds covalently to DNA to inhibit synthesis of DNA as with Carboplatin.

When Carboplatin is used as an agent for enhancing production of viral vectors, the concentration thereof in a medium in which viral vector-producing cells are cultured is not particularly limited. For example, the concentration can be 5 to 150 µg/ml. The concentration is preferably 10 to 100 µg/ml, more preferably 10 to 50 µg/ml, and further preferably 20 to 40 µg/ml.

As the DNA replication inhibitor, use of Cisplatin having the structure indicated below or an analog thereof is preferable.

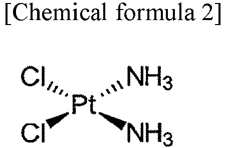

An analog of Cisplatin is a compound having a structure derived from the structure indicated above by substitution of a given atom or atoms with another atom or other atoms, which binds covalently to DNA to inhibit synthesis of DNA as with Cisplatin.

When Cisplatin is used as an agent for enhancing production of viral vectors, the concentration thereof in a medium in which viral vector-producing cells are cultured is not particularly limited. For example, the concentration can be 1 to 10 µg/ml. The concentration is preferably 1 to 5 µg/ml, and more preferably 1 to 3 µg/ml.

As the DNA replication inhibitor, in addition, use of Nedaplatin having the structure indicated below or an analog thereof is preferable.

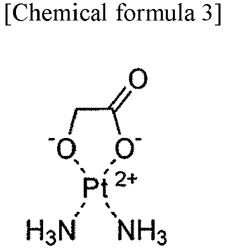

An analog of Nedaplatin is a compound having a structure derived from the structure indicated above by substitution of a given atom or atoms with another atom or other atoms, which binds covalently to DNA to inhibit synthesis of DNA as with Nedaplatin.

When Nedaplatin is used as an agent for enhancing production of viral vectors, the concentration thereof in a medium in which viral vector-producing cells are cultured is not particularly limited. For example, the concentration can be 1 to 20 µg/ml. The concentration is preferably 2 to 10 µg/ml, more preferably 2 to 7 µg/ml, and further preferably 2 to 5 µg/ml. Examples

Hereafter, the present invention is described in greater detail with reference to the examples, although the technical scope of the present invention is not limited to the examples below.

### [Method of experiment]

### 1) Cell culture

The cells of the immortalized cell line derived from the human embryonic kidney (HEK293 cells) purchased from ATCC were cultured in E-MEM supplemented with 10% fetal bovine serum, penicillin, and streptomycin at 37°C in the presence of 5% CO₂.

### 2) Preparation of AAV type 1 vector (rAAV1)

The recombinant adeno-associated virus 1 (rAAV1) was prepared with the use of 3 types of plasmids: pAAV-ZsGreen1 (TaKaRa Bio), pAAV2/1, and pHelper. A mixture of 3 types of plasmids for preparation of rAAV1 was adjusted to bring the mixing ratio of DNA to Polyethylenimine Max (PEI: Polysciences) (DNA:PEI) to 1:2 in OPTI-MEM, and the reaction was allowed to proceed at room temperature for 15 minutes. After 90% or higher confluent HEK293 cells were transfected with E-MEM supplemented with 10% FBS for 6 hours, culture was performed in serum-free E-MEM to prepare the AAV type 1 vector.

### 3) Measurement of virus titer by quantitative PCR (qPCR)

Genome DNA of rAAV1 was extracted to measure the virus titer. Specifically, the culture supernatant was collected and treated with benzonase at 37°C for 1 hour, and the viral genome DNA was extracted and purified using the DNeasy Blood & Tissue kit (QIAGEN). The virus titer was measured by real-time PCR in triplicate. qPCR was performed using primers targeting Zs-Green 1.

### 4) Evaluation of AAV vector-producing ability by treatment with chemical substance

The HEK293 cells were seeded at 4 × 10⁵ cells/well on a 24-well plate, plasmids for preparation of AAV vectors were introduced on the following day, the transfection solution was removed 6 hours later, the medium was exchanged with serum-free E-MEM supplemented with a DNA replication inhibitor, such as Carboplatin, Cisplatin, or Nedaplatin, at various concentrations as shown in Table 1, and culture was then performed for 3 days. The virus titers achieved with the use of the chemical substances were compared with the virus titer of the control cells treated with serum-free E-MEM not supplemented with chemical substances. Changes in the amount of AAV production of Carboplatin were observed with the elapse of time for 4 to 7 days.

**[Table 1]**

| Chemical substance used | Manufacturer | Concentration |
|---|---|---|
| Carboplatin | Wako | 10, 20, 30, 40, 50, 100 µg/ml |
| Cisplatin | Fujifilm | 1, 2, 5, 10, 20 µg/ml |
| Nedaplatin | Selleckchem.com | 2, 5, 10, 25 µg/ml |

### 5) Evaluation of AAV vector quality by treatment with chemical substance

The quality of the AAV vectors produced from the HEK293 cells treated with chemical substances was evaluated in terms of purity and the infectious ability by SDS-PAGE.

### 1. Measurement of purity by SDS-PAGE

The HEK293 cells were cultured to 90% or higher confluency in 2 square dishes. The HEK293 cells were transfected with plasmids for preparation of AAV vectors. The medium was exchanged with serum-free E-MEM supplemented with Carboplatin (20 µg/ml, 72 ml) 6 hours later, and culture was then performed for 5 days. The HEK293 cells that were not treated with chemical substances were employed as the control cells. The cells were collected using a scraper 5 days after the initiation of culture. The cells were centrifuged at 2000g for 10 minutes, and the AAV vectors were extracted, purified, and concentrated using the AAVpro Purification Kit (TaKaRa Bio). Thereafter, the virus titers were measured by qPCR, and SDS-PAGE was performed at 6.1 × 10⁹ v.g./lane on 5% to 20% polyacrylamide concentration-gradient gel. Following the electrophoresis, gel was stained using Oriole Fluorescent Gel Stain (Bio-RAD), the image of the major bands of the AAV capsid proteins (VP1, VP2, and VP3) was obtained using a chemiluminescence imaging system, and the proportion (1:1:10) and the purities of the bands were analyzed.

### 2. Evaluation of infectious ability

The HEK293 cells were seeded at 4 × 10⁵ cells/well on a 24-well plate, plasmids for preparation of AAV vectors were transfected thereinto, and the resultant was then treated with chemical substances for 5 days. The culture supernatant was then collected. The HEK293 cells that were not treated with chemical substances were employed as the control cells. The virus titers were measured by qPCR, the HEK293 cells seeded at 6.25 × 10³ cells/well on a 96-well plate were transduced at 1 × 10⁵ v.g./cell, and, 3 days thereafter, a green fluorescence was observed and the image thereof was obtained under an inverted research microscope.

### [Results of experiment]

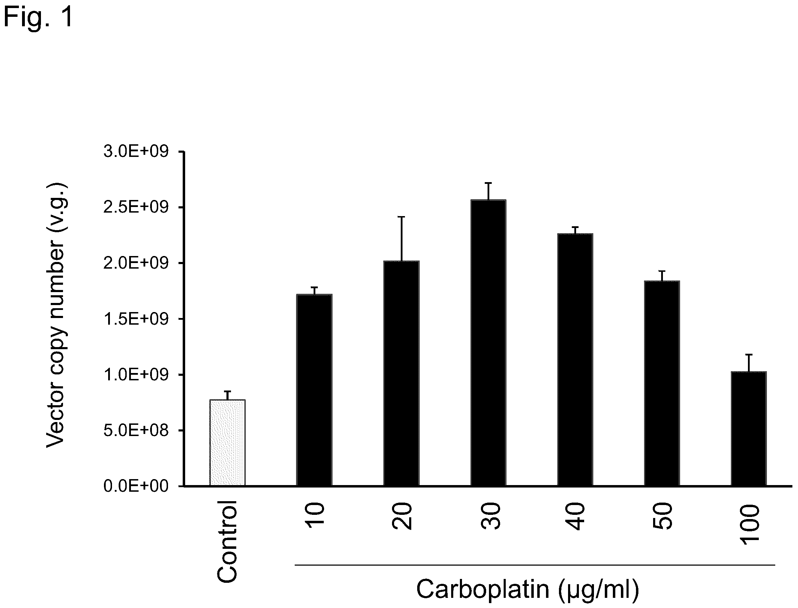

Figure 1 shows the results of measuring the effects of enhancing production of rAAV vectors achieved with the use of Carboplatin. In Figure 1, the vertical axis represents the amount of production of AAV vectors (v.g.), and the horizontal axis represents the concentration of the chemical substance. As shown in Figure 1, Carboplatin has effects of enhancing the amount of production of AAV vectors, and, in particular, greater effects of enhancing production of AAV vectors are observed in a range of concentration from 20 to 40 µg/ml.

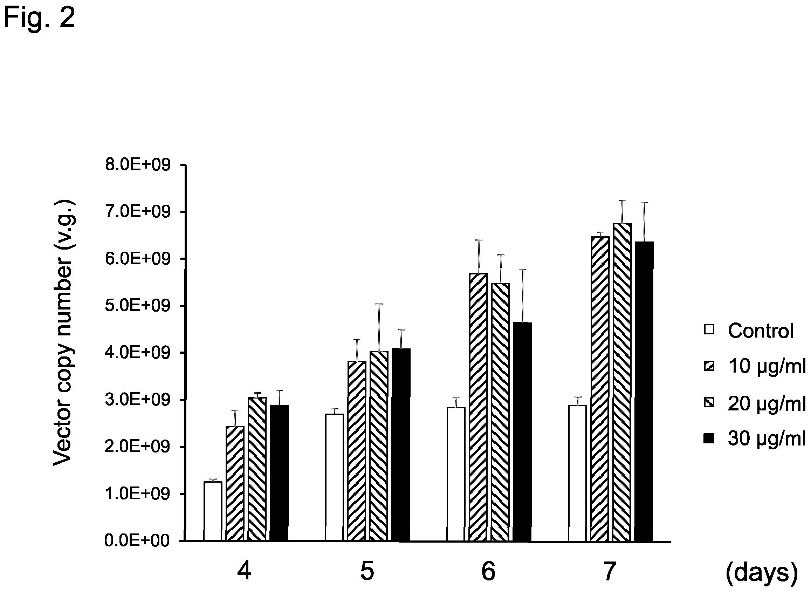

Figure 2 shows the results of measuring the effects of enhancing production of rAAV vectors achieved with the use of Carboplatin with the elapse of time. In Figure 2, the vertical axis represents the amount of production of AAV vectors (v.g.), and the horizontal axis represents the time elapsed after the initiation of vector production (days). As shown in Figure 2, the amount of production of AAV vectors is increasing with the elapse of time after the initiation of vector production, and, in particular, the amount of production of AAV vectors is increasing 7 days after the treatment.

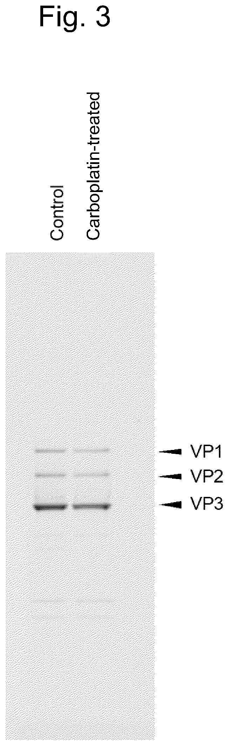

Figure 3 shows the results of the SDS-PAGE analysis of rAAV vector-constituting proteins when Carboplatin is used. As shown in Figure 3, the rAAV vector produced by treatment with Carboplatin comprises rAAV-constituting proteins (VP1, VP2, and VP3) equivalent to those of the rAAV vector prepared without treatment.

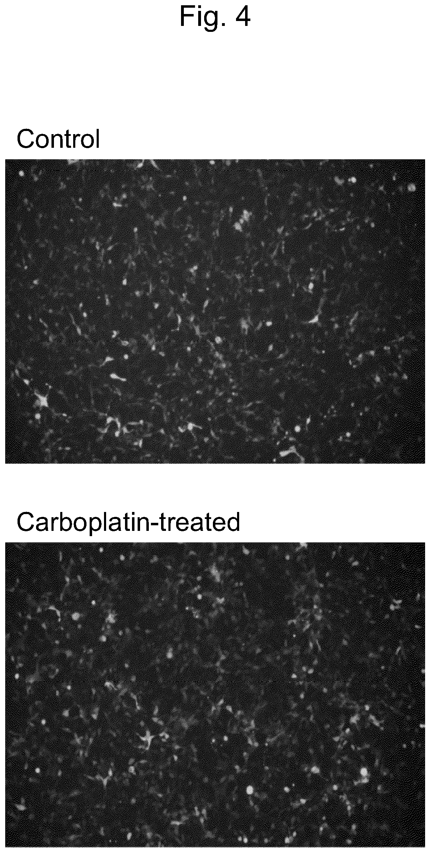

Figure 4 shows the results of the infectivity test of the rAAV vectors produced with the use of Carboplatin. As shown in Figure 4, the rAAV vector produced by treatment with Carboplatin retains the infectious ability equivalent to that of the AAV vector prepared without treatment with Carboplatin.

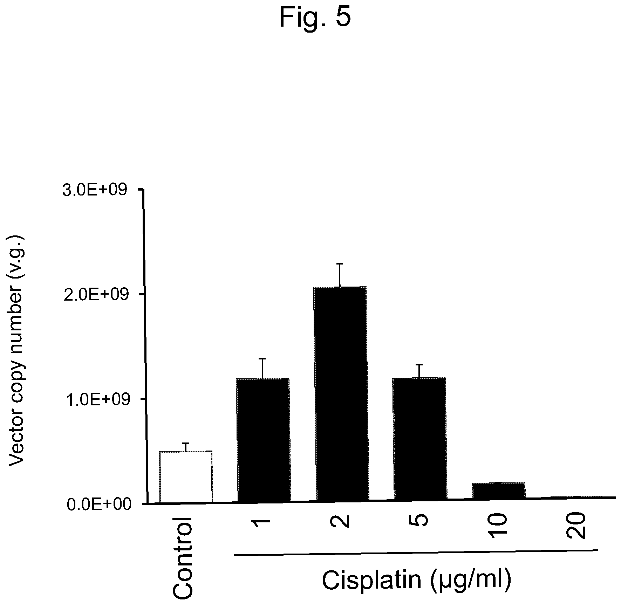

Figure 5 shows the results of measuring the effects of enhancing production of rAAV vectors achieved with the use of Cisplatin that inhibits DNA replication in the same manner as with Carboplatin. In Figure 5, the vertical axis represents the amount of production of AAV vectors (v.g.), and the horizontal axis represents the concentration of the chemical substance. As shown in Figure 5, Cisplatin has effects of enhancing the amount of production of AAV vectors as with Carboplatin, and, in particular, greater effects of enhancing production of AAV vectors are observed in a range of concentration from 1 to 5 µg/ml.

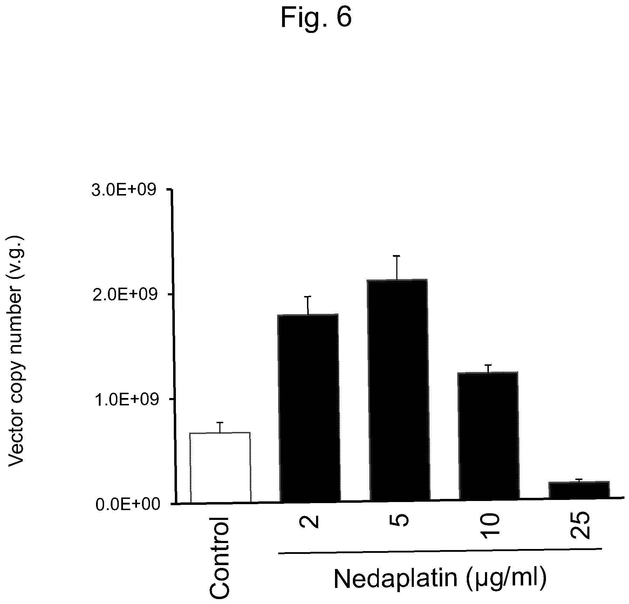

Figure 6 shows the results of measuring the effects of enhancing production of rAAV vectors achieved with the use of Nedaplatin that inhibits DNA replication. In Figure 6, the vertical axis represents the amount of production of AAV vectors (v.g.), and the horizontal axis represents the concentration of the chemical substance. As shown in Figure 6, Nedaplatin has effects of enhancing the amount of production of AAV vectors as with Carboplatin, and, in particular, greater effects of enhancing production of AAV vectors are observed in a range of concentration from 2 to 10 µg/ml.

The results demonstrate that the effects of enhancing production of rAAV vectors can be achieved by allowing a DNA replication inhibitor, such as Carboplatin, Cisplatin, or Nedaplatin, to act on the viral vector-producing cells.

## Claims

1. An agent for enhancing production of a viral vector comprising, as a major ingredient, a DNA replication inhibitor.

2. The agent for enhancing production of a viral vector according to claim 1, which is used for production of a viral vector using a viral vector-producing cell.

3. The agent for enhancing production of a viral vector according to claim 1, wherein the DNA replication inhibitor is a platinum-based drug.

4. A method for producing a viral vector comprising: a step of culturing a viral vector-producing cell in which DNA replication is inhibited; and a step of collecting the viral vector from the cultured cell.

5. The method for producing a viral vector according to claim 3, wherein, in the step of culturing the cell, the cell is cultured in a medium supplemented with the agent for enhancing production of the viral vector according to any of claims 1 to 3.
